# EUROPEAN PATENT APPLICATION

(11) **EP 4 632 757 A1**
(43) Date of publication of application: **15.10.2025**
(21) Application number: 24169275.5
(22) Date of filing: 09.04.2024
(51) Int. Cl.: G16H 30/20, G16H 30/40, G16H 40/63, G16H 50/20

(54) **POSE CORRECTION DEVICE AND METHOD FOR MEDICAL DIAGNOSTIC AND TREATMENT**

(71) Applicant: Siemens Healthineers AG, 91301 Forchheim (DE)
(72) Inventor: The designation of the inventor has not yet been filed
(74) Representative: Siemens Healthineers Patent Attorneys

(57) **Abstract**

The invention relates to a method for correcting a current pose (28) of a patient (26) for a diagnostic image capture procedure, comprising: receiving (104) an input image of the patient (26); determining (102), by means of a first artificial neural network, a target patient pose (22) from a positioning goal (18); determining (106) a current pose (28) of the patient from the input image and determining (108) a correction plan (30) for correcting the pose of the patient (26) from the current patient pose (28) and the target patient pose (22). Furthermore, the invention relates to a device, a computer program product and a computer-readable medium comprising instructions for carrying out the method.

## Description

The invention relates to methods and systems for preparing a patient for diagnostic imaging and/or therapy in a medical context.

Patient positioning forms one of the most important steps of any diagnostic imaging or therapy workflow.

In diagnostic imaging, the correct positioning of the patient determines whether the intended biological structures will be usefully captured by a diagnostic imaging device. When the patient is not correctly positioned, medical conditions may not be shown clearly in the resulting image. This may result in the necessity for re-takes or sub- optimal or wrong diagnoses. Furthermore, depending on the medical imaging device, certain positions of body parts of the patient may result in better image quality than others.

Performing an effective physical therapy often requires the patient to be in a suitable position. Errors in such positions may result in ineffective therapies.

Contrary to its importance, patient positioning is also one of the most manual in subjective parts of most diagnostic imaging art therapy workflows. Thus, the quality of the positioning heavily depends on the experience of the executing technician. Consequently, patient positioning is both time-consuming and error prone.

Repositioning aids are known for consistently repositioning a patient between two instances, for example during a planning CT and then during radiation therapy. However, these repositioning aids assume that the patient was positioned optimally in the first place.

In patient positioning, the collection of positions and orientations of all movable parts of the patient body are referred to as the patient pose.

In light of the above, the present invention provides methods and devices that help to improve the quality and results of medical image and therapeutic workflows and increase the degree of automation of said workflows. The present invention aims to guide its users, for example technicians and/or patients, to correct a pose in preparation of diagnostic imaging and/or therapy.

To this end, a computer-implemented method, a data processing device, a computer program product and a computer-readable medium according to the independent claim are proposed. Advantageous embodiments of the invention are the subject of the dependent claims.

To reach the above-mentioned goals, a computer-implemented method for correcting a current pose of a patient is proposed, comprising: receiving an input image of the patient; determining, by means of a first artificial neural network, a target patient pose from a positioning goal; determining a current pose of the patient from the input image and determining a correction plan for correcting the pose of the patient.

In this way, the quality of the positioning of the patient is no longer entirely dependent on the knowledge and skill of the technician or therapy worker.

In some embodiments, the positioning goal comprises a diagnostic or therapeutic scenario or question.

Such artificial neural networks can be trained from annotated training data taken from previous cases. In this way, the experience of many technicians and/or therapeutic workers can be incorporated into the method.

In some embodiments, the method comprises the step evaluating whether the current patient pose is close to the target patient pose, wherein the previously mentioned steps are repeated until the poses are evaluated to be close.

In this way, continuous feedback may be given to the patient or technician on how to reach the target patient pose until the target patient pose is reached. The patient technician then knows that the position is correct.

In some embodiments, the step of evaluating comprises calculating, from a difference of parameters of the current patient pose and of the target patient pose, a conformity indicator and evaluating the poses to be close if the conformity indicator passes a threshold value.

Depending on the diagnostic or therapeutic procedure in question, the threshold can be set to more or less strict values to take into account the degree of precision required for any particular procedure. That way the position can be correct without being overly pedantic to the technician and/or patient.

In some embodiments, the method comprises the step converting the correction plan into a textual correction feedback by means of an artificial neural network.

Such a textual correction feedback may, for example, be displayed to a user for self-positioning or to a technician to aid with correcting the pose of the patient. Textual representations may also be fed to speech synthesis software to be read out so that the technician can keep his eyes on the patient while receiving the correction plan.

In some embodiments, the method comprises the step converting the correction plan into a pose correction animation showing how the pose of the patient should be modified.

In some instances, such as when a patient is supposed to self-correct the pose, it may be easier and clearer to show the intended movement of the body towards the target position. Such a pose correction animation may also be easily intelligible by patients who do not understand the system language or language in general.

In some embodiments, the pose correction animation is created from pose parameters obtained by linear interpolation between parameters of the current patient pose and the target patient pose.

This is a very simple way to create a smooth animation.

In some embodiments, the correction plan is determined to comprise a correction of a spatial position of the patient.

If the patient is correctly posed but not at the right position relative to the medical diagnostic instrument, the positioning error can be detected in this way. This may, for example, allow the patient to be arranged so that the diagnostic image quality obtainable is maximized.

In some embodiments, the step determining the target patient pose from a positioning goal is carried out by means of an artificial neural network trained using synthetic pose data generated from human kinetic models and/or using natural language databases and/or medical protocol definition databases.

Training data augmented with such synthetic pose data allows generating an unlimited number of patient poses to train the artificial neural network with. Both the recognition of the current pose as well as the determination of the correction plan are improved and made more robust by the use of such augmented training data.

In some embodiments, at least one of the method steps is carried out by means of an artificial neural network.

The expertise the artificial neural network was trained with is thus added to the preparation of the medical imaging or therapy session. Accordingly, rare medical imaging situations and rare therapeutic situations are more reliably handled, improving the quality of the imaging and/or therapeutic workflow.

In some embodiments, the step of determining a target patient pose from a positioning goal is carried out by means of a large language model.

Such models are particularly well suited to transforming text into other forms of information.

The problem is also solved by a data processing device comprising means for carrying out the method according to any of the method claims.

The problem is also solved by a computer program product comprising instructions which, when the program is executed by a computer, cause the computer to carry out the methods of any of the method claims.

The problem is also solved by a computer-readable data carrier having stored thereon said computer program product.

Further embodiments and advantages may be gathered from the enclosed figures which schematically show embodiments of the invention. In particular:
- Fig. 1: shows a schematic representation of a pose correction determination device according to an embodiment of the invention comprised in a medical imaging environment;
- Fig. 2: shows a flow diagram of a method for correcting a current pose of a patient according to an embodiment of the invention;
- Fig. 3: shows a representation of an example pose correction animation And
- Fig. 4: shows a high-level block diagram of a computer that may be used to implement one or more embodiments.

The present invention generally relates to methods and systems for preparing a patient for diagnostic imaging and/or therapy in a medical context. In particular, the present invention relates to methods and systems for correcting a pose of a patient. Embodiments of the present invention are described herein to give a visual understanding of such methods and systems.

A digital image is often composed of digital representations of one or more objects (or shapes). The digital representation of an object is often described herein in terms of identifying and manipulating the objects. Such manipulations may be virtual manipulations accomplished in the memory or other circuitry/hardware of a computer system. Accordingly, is to be understood that embodiments of the present invention may be performed within a computer system using data stored within the computer system.

A pose of a patient may be represented in a computer system in a variety of ways. In some embodiments, the pose may be represented as a collection of landmarks, for example joints and/or bones, of the patient body. As a template for storing a pose in a memory or storage device of a computer, a pose model may be defined, comprising the relevant landmarks.

For example, for each joint from the pose model, an orientation of the attached body parts such as bones relative to each other may be stored together with their relation to each other. A knee joint of a healthy human leg will, for example, have a thigh and calf attached, which enclose an angle between them. An angle of 180° would indicate a stretched knee whereas an angle of 90° would indicate a knee bent such that thigh and calf would form a right angle.

When defining the pose model, care should be taken to include and model all relevant landmarks, e.g. joints, together with their degrees of freedom. The pose model does not need to be anatomically correct in its entirety as long as the observable degrees of freedom are respected. Between foot and knee, for example, the tibia and fibula provide an observable hinge around which the foot can turn relative to the thigh. While the anatomic reality behind this movement is more complicated, it is, for the purpose of pose correction, sufficient to model the observable hinge.

The pose model may, in some embodiments, further include distances between landmarks to account for differently built patients. Also, in some embodiments, the pose model may include spatial positions of body parts such as major bones.

The pose model may, in some embodiments, further comprise data regarding a spatial localization such as a position and/or orientation of the patient relative to another component, for example relative to a diagnostic table, relative to an active diagnostic area of an instrument or to another object.

Embodiments described herein provide for correcting a pose of a patient in preparation of diagnostic imaging and/or therapy. Embodiments described herein use semantic knowledge of optimized poses, medical imaging and therapeutic measures, to detect and correct patient poses that are ill-suited to the next diagnostic or therapeutic step. Further, embodiments described herein use such semantic knowledge to provide pose correction information to the patient, medical personnel and/or medical equipment to improve the pose of the patient.

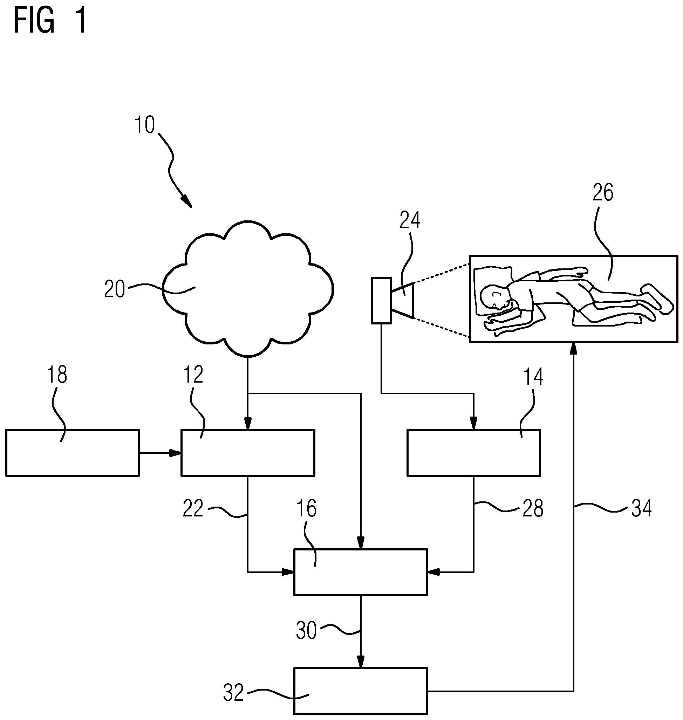

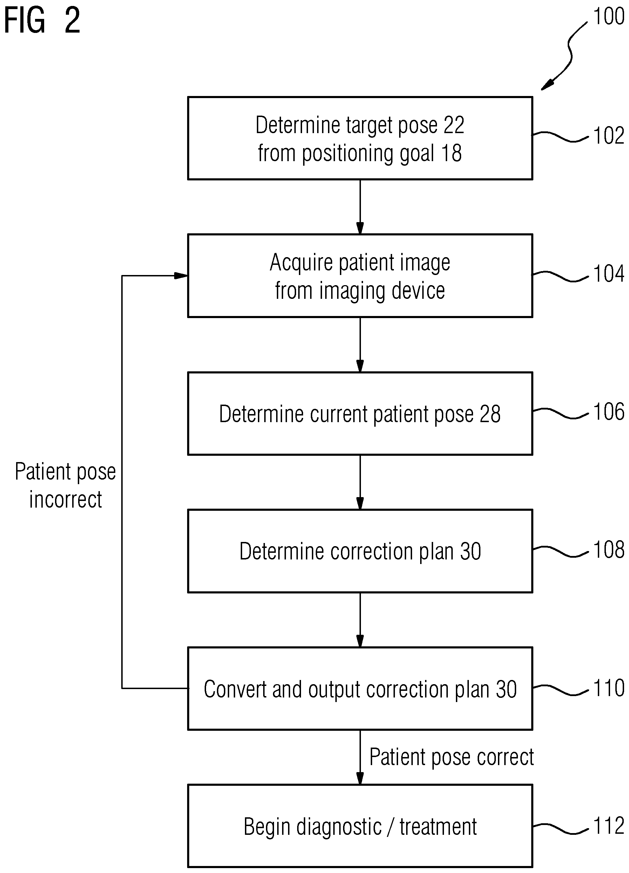

A pose correction determination device 10 as shown in **Fig. 1** is configured to carry out steps of a method 100 as shown in **Fig. 2** and comprises a target pose determination device 12, A current pose determination device 14 and a correction plan determination device 16.

The target pose determination device 12 comprises a first artificial neural network such as a large language model (LLM) which is trained to identify an optimum patient position for a given diagnostic question or therapy target. The diagnostic question or therapy target may be input as a positioning goal 18. A positioning goal 18 may be, for example, formulated as text such as "lateral X-Ray of left knee joint" or as a diagnostic question such as "Are the ligaments in the left knee joint intact?". In some embodiments, as an aid to forming diagnostic questions, a collection of frequently used diagnostic procedures may be provided for selection on a human interface device (HID).

In some embodiments, a medical and anatomical knowledge database 20 is provided which may be used for training the artificial neural network or may be available as a supplementary input during operation.

Guidelines for diagnostic procedures, describing preparations step by step, exist and may be used to expand on the diagnostic question as well as within the medical and anatomical knowledge database 20.

From these inputs, i.e. the diagnostic question or the therapeutic target and, if present, the database 20, the target pose determination device 12 determines a target patient pose 22 in a first method step 102. As noted above, the target patient pose 22 may be based on the pose model as described above..

An imaging device, such as a camera 24 is provided to perceive a patient 26 and, in a method step 104, record images of the patient 26. The camera 24 may be any optical sensor providing a digital representation of the recorded scene which contains the patient 26. The imaging device may, for example, be a simple RGB camera recording in the visual spectrum, an infrared camera or a thermal imaging device. In some embodiments, the imaging device may be or comprise a 3D optical sensor. In some cases, the camera 24 may be a network camera and the recorded images may be provided as a video stream over a computer network.

The one or more images recorded by the camera 24 are provided to the current pose determination device 14 as input images for determining a current patient pose 28 in method step 106. The pose determination device 14 may comprise a second artificial neural network such as a perception module. Just as the target patient pose 22, the current patient pose 28 may be a digital representation of the actual position of the various movable parts of the body of the patient 26. Where such information is included in the pose model, the current patient pose 28 may comprise patient position and /or orientation data such as the position and/or orientation of the patient 24 on a diagnostic table and/or relative to the camera 24.

The target patient pose 22 and the current patient pose 28 are provided to the correction plan determination device 16 which determines a correction plan 30 in method step 108. The correction plan 30 may comprise one or more directives regarding how to change position of individual parts of the body of the patient 26 to be closer to the target patient pose 22. Each of the directives may, for example, describe a translation and/or rotation of a particular body part such as a bone or joint. In some cases, limits of the human body or physical interaction of moving body parts may require a sequence of such directives.

The correction plan determination device 16 may have access to the medical and anatomical knowledge database 20. Furthermore, the correction in determination device 16 may comprise one or more third artificial neural networks such as a large language model (LLM) and/or a perception model (FM). Artificial neural networks that combine both an LLM as well as an FM network may be particularly well-suited for this task. One example of such networks is called MotionGPT and will be elaborated further later in this document.

The correction plan 30 is provided to a user feedback device 32 for output in step 110. In some embodiments, the user feedback device 32 may, for example, be incorporated into the correction plan determination device 16. The user feedback device 32 may also, for example, comprise a fourth artificial neural network to transform the correction plan 30 into correction instructions 34.

Getting from a current patient pose 28 of the patient 26 to the target patient pose 22 may involve, for example, moving and/or rotating multiple limbs. Thus, the correction plan 30 will, in most cases, comprise multiple instructions relating to how to improve the patient pose.

In some embodiments, the artificial neural networks, if present, of the target pose determination device 12, the current pose detection device 14 and/or the correction plan determination device 16 may comprise a pose or motion tokenizer and/or encoder which will translate an observed or described pose or motion to an internal representation of the pose or motion, such as position or motion tokens or a collection of position or motion tokens, e.g. represented as vectors. This internal representation comprises the relevant aspects to define the motion and/or the pose. Artificial neural networks generally process data with such internal representations instead of images or text.

The internal representation may also be called an embedding. Such an embedding only makes sense within the confines of one artificial neural network. To reuse it in different artificial neural networks, it would usually need to be translated, decoded and/or encoded. In some embodiments it may thus be expedient to implement multiple devices comprising an artificial neural network as one or more linked artificial neural networks trained together and sharing the same embedding.

After processing, the internal representation, e.g. in this case the internal representation of the correction plan 30, needs to be translated back into a form intelligible by humans or other less advanced machines to be useful. This may, in this particular context, be achieved by use of a motion or pose decoder. The resulting correction plan 30 may then be further processed, for example as follows:
In some embodiments, the user feedback device 32 is configured to convert the correction plan 30 into correction instructions 34 comprising a textual description of instructions on how to reach the target patient pose 22. Such correction instructions 34 may, for example, be shown on a display for a technician or the patient. An example for a textual description of a correction plan 30 directed at a patient or technician may be: "Please flex the upper leg upwards and straighten the torso." In some embodiments, such instructions may also comprise instructions to move the patient 26 such as "move down on the diagnostic table by about 10 cm."

In some embodiments, the correction instructions 34 are redetermined every time the current patient pose 28 changes. In this way, the technician or the patient receives immediate feedback relating to the changes effected.

In some embodiments, the textual correction instructions 34 may be fed to a text-to-speech converter which reads the instruction to the technician or the patient. Such vocal instructions have the advantage that, in particular a technician, may receive the correction instructions 34 while looking at the patient 26.

When the current patient pose 28 is deemed close enough or equal to the target patient pose 22, the diagnostic or treatment is begun in method step 112.

How the decision whether the current patient pose 28 is deemed close enough or equal to the target patient pose 22 is reached depends on the implementation, in particular on the implementation of the pose model. For example, in embodiments in which the pose model comprises the angles of body parts at joints, a cumulative difference or a sum of squared differences over all the angles between the poses 22, 28 may be calculated as a conformity indicator. When the conformity indicator drops below an empirically obtainable threshold, the current patient pose 28 may be deemed close enough to the target patient pose 22.

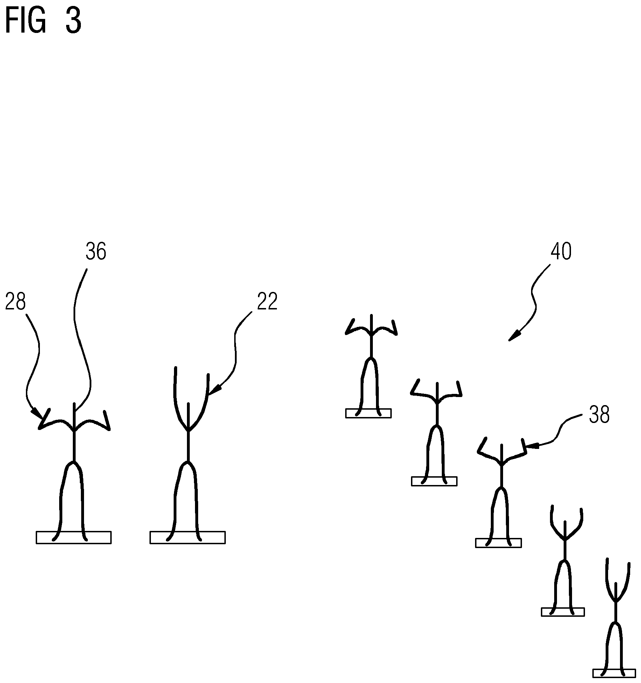

In some embodiments, the user feedback device 32 is configured to convert the correction plan 30 into a pose correction animation such as it is schematically shown in **Fig. 3****.** Such a pose correction animation may, for example, be shown to the technician of the patient on a display. The current patient pose 28 and/or the target patient pose 22 may, for example, be represented as a stick figure 36, showing the position of a major bone is within the body of the patient 26. The pose correction animation will then comprise a multitude of intermediate representations 38 which, when displayed one after another, show the movement that needs to be affected by the patient or the technician to get the body from the current patient pose 28 to the target patient pose 22.

In some embodiments, the stick figure 36 may be created by drawing a projected image of the pose model, wherein the stick figure 36 is drawn with the joints and other body parts comprised in the pose model represented, for example, as lines, circles or other geometric forms.

The multitude of intermediate representations 38 may, in simple cases, be acquired by linear interpolation between the current patient pose 28 and the target patient pose 22. Applied to their digital representation as pose models in particular, said linear interpolation may be applied to the angles between body parts. In this way, a smooth motion of the animation may be obtained.

In more complex scenarios, linear interpolation may not be possible because of physical limitations. Linear interpolation may, in particular, not take physical collisions into account or lead to overextension of joints. For example, the human leg easily bends backward but not forward since the knee joint only has a limited range of movement which is not taken into account by linear interpolation. In such scenarios, more complex models may be required, such as LLMs with pose understanding.

In such complex scenarios, it may be necessary that body parts may not be moved together at the same time but that a sequence of corrective movements needs to be established. Accordingly, the resulting pose correction animation may comprise multiple parts, each corresponding to movements that may be effected simultaneously.

If the pose is correct, the correction instructions 34 may indicate that the pose is correct and that no correction is needed.

In some embodiments, to train the perception model comprised in the current pose determination device 14, image data which has been annotated by experts may be used. Such annotations may, for example, comprise indications of the position and orientation of certain joints and/or bones or other body parts in each annotated image such that the perception model may later identify each bone and may determine the position and/or orientation of said bone within an image. The perception model may, for example, comprise any kind of human pose detection model known, including but not limited to image-to-image models such as U-Net or Dense-U-net when regressing heat maps, or more complex multi-step architecture such as motionBERT (https://arxiv.org/pdf/2210.06551v5.pdf).

In some embodiments, to train the first, second, third and/or fourth artificial neural networks of the target pose determination device 12, the correction plan determination device 16 and/or the user feedback device 32, existing databases of natural language and pose understanding may be used. Furthermore, the artificial neural networks may be finetuned on medical protocol definitions. Also, in some embodiments, institution such as hospitals, may have developed their proper positioning protocols for some or all imaging and/or therapy situations, which may be used to fine-tune the models. In some embodiments, synthetic pose data can be generated from human kinematic models and be used for further training the large language models. In some embodiments, the large language model may be a MotionGPT model (https://arxiv.org/pdf/2306.14795.pdf).

In some embodiments one or more of the first, second, third and fourth artificial neural networks may be combined to form one artificial neural network.

The pose correction determination device 10 may be implemented as a data processing device such as a computer. It is thus to be understood that elements that have been described as devices, such as, for example, the target pose determination device 12, the current pose determination device 14 and the correction plan determination device 16, may be implemented as modules of a computer program running on said data processing device.

More generally, systems, devices, and methods described herein may be implemented using digital circuitry, or using one or more computers using well-known computer processors, memory units, storage devices, computer software, and other components. Typically, a computer includes a processor for executing instructions and one or more memories for storing instructions and data. A computer may also include, or be coupled to, one or more mass storage devices, such as one or more magnetic disks, internal hard disks and removable disks, magneto-optical disks, optical disks, etc.

Systems, devices, and methods described herein may be implemented using computers operating in a client-server relationship. Typically, in such a system, the client computers are located remotely from the server computer and interact via a network. The client-server relationship may be defined and controlled by computer programs running on the respective client and server computers.

Systems, devices, and methods described herein may be implemented within a network-based cloud computing system. In such a network-based cloud computing system, a server or another processor that is connected to a network communicates with one or more client computers via a network. A client computer may communicate with the server via a network browser application residing and operating on the client computer, for example. A client computer may store data on the server and access the data via the network. A client computer may transmit requests for data, or requests for online services, to the server via the network. The server may perform requested services and provide data to the client computer(s). The server may also transmit data adapted to cause a client computer to perform a specified function, e.g., to perform a calculation, to display specified data on a screen, etc. For example, the server may transmit a request adapted to cause a client computer to perform one or more of the steps or functions of the methods and workflows described herein, including one or more of the steps or functions of Figures 1 or 2. Certain steps or functions of the methods and workflows described herein, including one or more of the steps or functions of Figures 1 or 2, may be performed by a server or by another processor in a network-based cloud-computing system. Certain steps or functions of the methods and workflows described herein, including one or more of the steps of Figures 1 or 2, may be performed by a client computer in a network-based cloud computing system. The steps or functions of the methods and workflows described herein, including one or more of the steps of Figures 1 or 2, may be performed by a server and/or by a client computer in a network-based cloud computing system, in any combination. Systems, devices, and methods described herein may be implemented using a computer program product tangibly embodied in an information carrier, e.g., in a non-transitory machine-readable storage device, for execution by a programmable processor; and the method and workflow steps described herein, including one or more of the steps or functions of Figures 1 or 2, may be implemented using one or more computer programs that are executable by such a processor. A computer program is a set of computer program instructions that can be used, directly or indirectly, in a computer to perform a certain activity or bring about a certain result. A computer program can be written in any form of programming language, including compiled or interpreted languages, and it can be deployed in any form, including as a stand-alone program or as a module, component, subroutine, or other unit suitable for use in a computing environment.

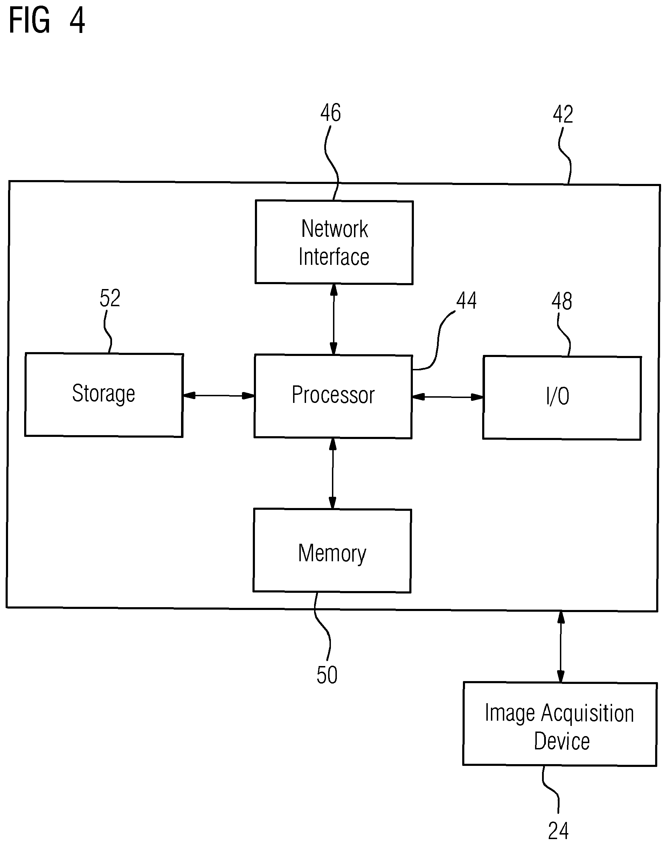

A high-level block diagram of an example computer 42 that may be used to implement systems, devices, and methods described herein is depicted in Fig. 4. Computer 42 includes a processor 44 operatively coupled to a data storage device 52 and a memory 50. Processor 44 controls the overall operation of computer 42 by executing computer program instructions that define such operations. The computer program instructions may be stored in data storage device 52, or other computer readable medium, and loaded into memory 50 when execution of the computer program instructions is desired. Thus, the method and workflow steps or functions of Figures 1 or 2 can be defined by the computer program instructions stored in memory 50 and/or data storage device 52 and controlled by processor 44 executing the computer program instructions. For example, the computer program instructions can be implemented as computer executable code programmed by one skilled in the art to perform the method and workflow steps or functions of Figures 1 or 2. Accordingly, by executing the computer program instructions, the processor 44 executes the method and workflow steps or functions of Figures 1 or 2. Computer 42 may also include one or more network interfaces 46 for communicating with other devices via a network. Computer 42 may also include one or more input/output devices 48 that enable user interaction with computer 42 (e.g., display, keyboard, mouse, speakers, buttons, etc.).

Processor 44 may include both general and special purpose microprocessors, and may be the sole processor or one of multiple processors of computer 42. Processor 44 may include one or more central processing units (CPUs), for example. Processor 44, data storage device 52, and/or memory 50 may include, be supplemented by, or incorporated in, one or more application-specific integrated circuits (ASICs) and/or one or more field programmable gate arrays (FPGAs).

Data storage device 52 and memory 50 each include a tangible non-transitory computer readable storage medium. Data storage device 52, and memory 50, may each include high-speed random access memory, such as dynamic random access memory (DRAM), static random access memory (SRAM), double data rate synchronous dynamic random access memory (DDR R_AM), or other random access solid state memory devices, and may include non-volatile memory, such as one or more magnetic disk storage devices such as internal hard disks and removable disks, magneto- optical disk storage devices, optical disk storage devices, flash memory devices, semiconductor memory devices, such as erasable programmable read-only memory (EPROM), electrically erasable programmable read-only memory (EEPROM), compact disc read-only memory (CD-ROM), digital versatile disc read-only memory (DVD-ROM) disks, or other non-volatile solid state storage devices.

Input/output devices 48 may include peripherals, such as a printer, scanner, display screen, etc. For example, input/output devices 48 may include a display device such as a cathode ray tube (CRT) or liquid crystal display (LCD) monitor for displaying information to the user, a keyboard, and a pointing device such as a mouse or a trackball by which the user can provide input to computer 42.

An image acquisition device 54 can be connected to the computer 42 to input image data (e.g., medical images) to the computer 42. It is possible to implement the image acquisition device 54 and the computer 42 as one device. It is also possible that the image acquisition device 54 and the computer 42 communicate wirelessly through a network. In a possible embodiment, the computer 42 can be located remotely with respect to the image acquisition device 54.

Any or all of the systems and apparatus discussed herein may be implemented using one or more computers such as computer 42.

One skilled in the art will recognize that an implementation of an actual computer or computer system may have other structures and may contain other components as well, and that Fig. 4 is a high level representation of some of the components of such a computer for illustrative purposes.

Designations such as "first", "second" or "third" are used in this document only to distinguish similar but different items. They do not designate any kind of hierarchy.

Independent of the grammatical term usage, individuals with male, female or other gender identities are included within the term.

The present invention provides a fundamentally different and novel approach to patient positioning. Firstly, it does no longer rely on the experience of the user. Rather, it leverages the vast medical and/or anatomical knowledge comprised in its training to determine an optimized pose for a given diagnostic question or therapy target automatically, without user interaction.

Furthermore, it allows for a pro-active feedback loop to the user for improving the pose before the actual diagnostic or therapy. It thus improves therapy efficiency and reduces the number of re-takes of images and, in the end, reduces misdiagnoses and ineffective therapies.

Last but not least, when feedback is given directly to a patient, a new "self-imaging" paradigm is allowed.

## Claims

1. A computer-implemented method (100) for correcting a current pose (28) of a patient (26) for a diagnostic image capture procedure, comprising:
receiving (104) an input image of the patient (26);
determining (102), by means of a first artificial neural network, a target patient pose (22) from a positioning goal (18);
determining (106) a current pose (28) of the patient from the input image and
determining (108) a correction plan (30) for correcting the pose of the patient (26) from the current patient pose (28) and the target patient pose (22).

2. The method of claim 1, comprising the step evaluating whether the current patient pose (28) is close to the target patient pose (22), wherein the steps according to claim 1 are repeated until the poses (22, 28) are evaluated to be close.

3. The method of claim 2, wherein evaluating comprises calculating, from a difference of parameters of the current patient pose (28) and of the target patient pose (22), a conformity indicator;
evaluating the poses (22, 28) to be close if the conformity indicator passes a threshold value.

4. The method of any of the previous claims, wherein the positioning goal (18) comprises a diagnostic or therapeutic scenario or question.

5. The method of any of the previous claims, comprising the step
converting (110) the correction plan (30) into a textual correction feedback (34).

6. The method of any of the previous claims, comprising the step
converting the correction plan (30) into a pose correction animation (40) showing how the pose of the patient (26) should be modified.

7. The method of claim 6, wherein the pose correction animation (40) is created from pose parameters obtained by linear interpolation between parameters of the current patient pose (28) and the target patient pose (22) .

8. The method of any of the previous claims, wherein the correction plan (30) is determined to comprise a correction of a spatial position of the patient (26).

9. The method of any of the previous claims, wherein the step determining (102) a target patient pose (22) from a positioning goal (18) is carried out by means of an artificial neural network trained using synthetic pose data generated from human kinetic models and/or using natural language databases and/or medical protocol definition databases.

10. The method of any of the previous claims, wherein at least another of the method steps (102, 106, 108, 110) is carried out by means of an artificial neural network.

11. The method according to any of the previous claims, wherein the step of determining (102) a target patient pose (22) from a positioning goal (18) is carried out by means of a large language model.

12. A data processing device (10) comprising means for carrying out the method (100) of any of the previous claims.

13. A computer program product comprising instructions which, when the program is executed by a computer, cause the computer to carry out the methods of any of the claims 1 to 11.

14. A computer-readable medium comprising instructions which, when executed by a computer, cause the computer to carry out the method of claim any of the claims 1 to 11.
